# EUROPEAN PATENT SPECIFICATION

(11) **EP 0 351 206 B1**
(45) Date of publication and mention of the grant of the patent: **14.09.1994**
(21) Application number: 89307070.6
(22) Date of filing: 12.07.1989
(51) Int. Cl.: A61M 25/00

(54) **A central venous catheter**
Zentralvenenkatheter
Cathéter pour la veine centrale

(30) Priority: 12.07.1988 US 217642
(43) Date of publication of application: 17.01.1990
(73) Proprietor: Cook Incorporated, Bloomington Indiana 47402 (US)
(72) Inventor: Cook, William A., Bloomington Indiana 47401 (US)
(74) Representative: Bannerman, David Gardner

(56) References cited:
- EP-A- 0 260 711
- US-A- 4 650 472
- US-A- 4 735 620

## Description

The present invention relates to the field of percutaneous catheterization and more particularly to a central venous catheter designed to decrease the incidence of erosion or perforation of the vasculature.

Central venous catheters have been used for several years to withdraw, inject or monitor fluids in the central vasculature. A recognized complication that can occur during the use of a central venous catheter is erosion or perforation of either the vena cava or atrium and subsequent cardiac tamponade. This complication is associated with substantial morbidity and mortality. Numerous guides have been developed which explain various procedures and techniques for proper insertion and placement of the central venous catheter. Although these guides set forth a multitude of warnings and precautions, the risk and incidence of complications associated with the central venous catheterization remains high.

One advance in this field involves the use of softer catheter materials. It appears soft catheter materials exhibit less of a tendency to erode and perforate the vasculature than stiffer materials. However, any percutaneously introducible material has the potential to perforate.

US-A-4650472, which concerns the percutaneous catheterisation of blood vessels, discloses a catheter having a curved tip for insertion into relatively-narrow blood vessels.

EP-A-0260711 describes a catheter for the treatment of aortic stenosis having a balloon for deflecting the leaflets of the aortic valve and a curved tip to avoid contact with the interior wall of the ventricle.

What is needed is a central venous catheter which reduces the risk and incidence of erosion and perforation of the vasculature.

Generally speaking, a catheter is provided for withdrawing, injecting or monitoring fluids within the central vasculature which tends to reduce the risk and incidence of erosion and perforation.

It is an object of the present invention to provide an improved central venous catheter which tends to decrease the risk and incidence of erosion and perforation of the vasculature.

According to the invention there is provided a central venous catheter for withdrawing, injecting or monitoring fluids within the central vasculature, comprising:
a one-piece flexible plastics tube of suitable diameter for percutaneous insertion into the vasculature, said tube having at least one lumen therethrough, the distal end of said tube being curved around at least 220° so as to present a blunt leading edge in use and when unobstructed by a guide wire.

Central venous catheters embodying the invention will now be described by way of non-limiting example and with reference to the accompanying drawings in which:
Fig. 1 is an elevational view of a catheter in accordance with the preferred embodiment of the present invention.
Fig. 2 is a cross-sectional view of the catheter of Fig. 1 taken along the lines 2--2 and shown in the direction of the arrows.
Fig. 3 is a cross-sectional view of a catheter tube of another embodiment of the present invention.

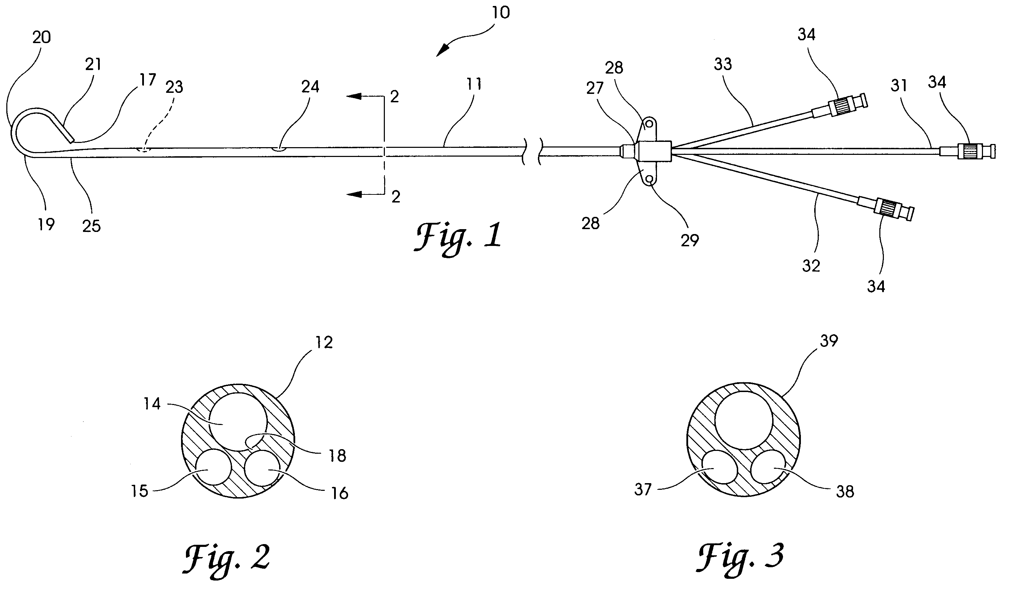

Referring to Fig. 1, there is shown a central venous catheter 10 in accordance with the preferred embodiment of the present invention. Catheter 10 includes a tube 11 having an external cross-section 12 as shown, for example, in Fig. 2. Tube 11 has three lumens 14, 15 and 16, each of which has a generally circular cross-section and is separated from an adjacent lumen by a wall portion such as is indicated at 18. Lumen 14 is generally larger than lumens 15 and 16 and is sized to receive a wire guide therethrough. In one specific embodiment of the invention, tube 11 is French Size 7, radiopaque polyurethane tubing. The diameter of lumen 14 is 0.109 cm (0.043 inches) and the diameter of both lumens 15 and 16 is 0.074 cm (0.029 inches). Such triple-lumen tubing is available commercially and may be purchased, for example, for Sabin Corporation of 617 South Curry Pike, P.O. Box 788, Bloomington, Ind. 47401 under the model designation UTLT-7.0.

The distal end 19 of catheter 10 is pigtailed. That is, catheter 10 is manufactured such that in its relaxed position, with its lumens unobstructed, distal end 19 curves around at least 220°, forming a partial loop. In the preferred embodiment, distal end 19 curves around about 235°. A portion 21 of distal end 19, nearest distal tip 17, is not curved and extends from the curved portion back toward the remainder of tube 11. The outer diameter of tube 11 at distal end 19 is slightly reduced from that of the remainder of tube 11. With distal end 19 in the curved position shown in Fig. 1, the leading "edge" is the blunt outer surface 20 of the pigtail. Moreover, due to the particular angle of curvature, distal end 19 provides only large, blunt surfaces (as opposed to the relatively pointed distal tip 17) for movement of catheter 10 in any direction within the vasculature.

Lumen 14 extends throughout the entire length of tube 11 and opens exteriorly at distal tip 17. A pair of sideports 23 and 24 are cut into the side of tube 11 as openings for lumens 15 and 16, respectively. Sideport 23 is approximately 2 cm ± 3 mm back from leading blunt surface 20 while sideport 24 is approximately 4 cm ± 3 mm back from blunt surface 20. Distal tip 17 and the point of transition 25 between constant diameter and the reduced diameter of distal end 19 are about 1.4 cm back from leading blunt surface 20.

Catheter 10 further includes a hub 27 having a pair of wings 28 and fixed to proximal end of tube 11. When catheter 10 is in use, wings 28 may be secured to the skin of the patient by taping or by suturing through apertures 29. Hub 27 has three passageways therethrough in alignment with and communicating with the three respective lumens of tube 11. Three extension tubes 31, 32 and 33 are fixed to and behind hub 27. Tube 31 is secured in alignment with and communication with the large diameter lumen 14 through the respective passageway in hub 27. Extension tubes 32 and 33 are likewise connected in alignment with the relevant passageways in hub 27 so as to be aligned with and in communication with smaller diameter lumens 15 and 16, respectively. Secured to the proximal ends of extension tubes 31, 32 and 33 are standard female Luer lock adapters 34. Such adapters are commercially available from Cook Incorporated, 925 South Curry Pike, Bloomington, Ind. 47402.

The catheter of the present invention is placed into the blood vessel by use of the procedure described in U.S. patent No. 4,650,472 and illustrated in Figs. 5a through 5e therein. U.S. patent No. 4,650,472 is hereby incorporated by reference. Catheter 10 of the present invention, and specifically tube 11, is made of a relatively flexible material which has insufficient rigidity to be advanced alone through the blood vessels. Further, pigtailed distal end 19 is too large to be introduced and advanced in the blood vessels in the coiled condition shown in Fig. 1. The procedure for inserting the catheter into the blood vessel generally involves a needle and a wire guide. After the needle has been inserted into the blood vessel, a wire guide is advanced through the needle and into the blood vessel until the distal end of the wire guide is positioned at the desired central venous site. While maintaining the wire guide in place, the needle is next withdrawn. As described and illustrated in Fig. 5d in U.S. patent No. 4,650,472, an inner cannula, appropriately sized to fit freely within lumen 14 of catheter 10 of the present invention, may be used with an appropriately sized wire guide. Catheter 10 of the present invention and the inner cannula (as shown in U.S. patent No. 4,650,472) are passed over the wire guide in unison and advanced with a twisting motion into the blood vessel until the distal end 19 of the catheter 10 nears the distal end of the wire guide and the desired central venous site. Alternatively, catheter 10 may be advanced directly over an appropriately sized wire guide without the aid of an inner cannula. Since tube 11 of catheter 10 is flexible, distal end 19 will be subsequently straight as it advances over the wire guide and through the blood vessel. Once catheter 10 is in place, the wire guide and inner cannula are withdrawn, leaving catheter 10 in place within the blood vessel.

With the wire guide and inner cannula removed, distal end 19 resumes its pigtail shape as shown in Fig. 1. The particular shape of the pigtail still allows distal end 19 to be moved freely within the central venous area as monitored by radiography. The pigtail design of the present invention, however, has exhibited a tremendous decrease in the tendency of the central venous catheter to cause erosion or perforation of the vasculature. With the wire guide and inner cannula removed, there are three open lumens providing access to the central venous site through extension tubes 31, 32 and 33.

Embodiments are also contemplated wherein the catheter tube defines fewer than three lumens.

In an alternative embodiment, Fig. 3 shows a cross-section of a catheter tube 39 where the smaller lumens 37 and 38 have an oval cross-section thereby increasing the volumetric flow potential therethrough.

While the invention has been illustrated and described in detail in the drawings and forgoing description, the same is to be considered as illustrative and not restrictive in character, it being understood that only the preferred embodiment has been shown and described and that all changes and modifications that fall within the scope of the claims are desired to be protected.

## Claims

1. A central venous catheter (10) for withdrawing, injecting or monitoring fluids within the central vasculature, comprising:
a one-piece flexible plastics tube (11) of suitable diameter for percutaneous insertion into the vasculature, said tube having at least one lumen (14) therethrough, the distal end (19) of said tube (11) being curved around at least 220° so as to present a blunt leading edge in use and when unobstructed by a guide wire.

2. The catheter for withdrawing, injecting or monitoring fluids of claim 1 wherein the distal end (19) of said tube (11) is tapered and said at least one lumen (14) extends completely through said tube, wherein said tube has a distal tip (17) and wherein a portion (21) of said distal end (19) adjacent said distal tip (17) is not curved.

3. The catheter for withdrawing, injecting or monitoring fluids of claim 1 or 2, wherein said tube (11) defines a second lumen (15) separate from said at least one lumen (14), said tube (11) also defining a first sideport (23) spaced from the distal end, and said second lumen (15) terminating at and being in communication with said first sideport (23).

4. The catheter for withdrawing, injecting or monitoring fluids of claim 3, wherein said tube defines a third lumen (16) separate from said at least one lumen (14) and from said second lumen (15), said tube (11) also defining a second sideport (24) spaced from the distal end (19) and from said first sideport (23), and said third lumen (16) terminating at and being in communication with said second sideport (24).

5. The catheter for withdrawing, injecting or monitoring fluids of claims 1,2,3 or 4, wherein the distal end (19) of said tube (11) curves around at least 235° when unobstructed by a guide wire.

6. The catheter of claim 1, wherein first lumen is disposed at the end of said distal tip (17).

7. The catheter of claim 6 wherein said sideport (23) of said second lumen (15) is proximal to said curved distal end (19).

8. The catheter of claim 7 wherein said sideport (24) of said third lumen (16) is proximal to said curved distal end (19).

9. The catheter of claim 8 wherein said second and third lumens (15,16) are spaced apart.

10. The catheter of claim 1 wherein the diameter of said tube (11) is reduced at said distal end.

11. The catheter of claim 1 wherein said curved distal end (19) of the tube (11) has a diameter smaller than the portion of the tube (11) that extends between the curved distal end and the proximal end.

## Patentansprüche

1. Ein zentraler Venenkatheter (10) zum Entnehmen, Injizieren oder Überwachen von Fluida innerhalb des zentralen Venenbereichs umfaßt:
ein einteiliges, flexibles Kunststoffrohr (11) mit zur perkutanen Einführung in den Venenbereich geeignetem Durchmesser, wobei das Rohr mindestens eine dadurch verlaufende Lichtung (14) aufweist und das distale Ende (19) des Rohrs (11) um mindestens 220° gebogen ist, um beim Gebrauch und wenn es unbehindert durch einen Führungsdraht ist, eine stumpfe Vorderkante darzustellen.

2. Der Katheter zum Entnehmen, Injizieren oder Überwachen von Fluida nach Anspruch 1, worin das distale Ende (19) des Rohrs (11) kegelförmig ist und sich die mindestens eine Lichtung (14) vollständig durch das Rohr erstreckt, worin das Rohr eine distale Spitze (17) aufweist und worin ein an die distale Spitze (17) angrenzender Bereich (21) des distalen Endes (19) nicht gebogen ist.

3. Der Katheter zum Entnehmen, Injizieren oder Überwachen von Fluida nach Anspruch 1 oder 2, worin das Rohr (11) eine von der mindestens einen Lichtung (14) separate zweite Lichtung (15) definiert, das Rohr (11) ebenfalls eine mit Abstand von dem distalen Ende angeordnete erste Seitenöffnung (23) definiert und die zweite Lichtung (15) an der ersten Seitenöffnung (23) endet und mit dieser in Verbindung ist.

4. Der Katheter zum Entnehmen, Injizieren oder Überwachen von Fluida nach Anspruch 3, worin das Rohr eine von der mindestens einen Lichtung (14) und von der zweiten Lichtung (15) separate dritte Lichtung (16) definiert, das Rohr (11) ebenfalls eine mit Abstand von dem distalen Ende (19) und von der ersten Seitenöffnung (23) angeordnete zweite Seitenöffnung (24) definiert und die dritte Lichtung (16) an der zweiten Seitenöffnung (24) endet und mit dieser in Verbindung ist.

5. Der Katheter zum Entnehmen, Injizieren oder Überwachen von Fluida nach Anspruch 1, 2, 3 oder 4, worin sich das distale Ende (19) des Rohrs (11) um mindestens 235° biegt, wenn es unbehindert durch einen Führungsdraht ist.

6. Der Katheter nach Anspruch 1, worin eine erste Lichtung an dem Ende der distalen Spitze (17) angeordnet ist.

7. Der Katheter nach Anspruch 6, worin die Seitenöffnung (23) der zweiten Lichtung (15) in der Nähe des gebogenen distalen Endes (19) ist.

8. Der Katheter nach Anspruch 7, worin die Seitenöffnung (24) der dritten Lichtung (16) in der Nähe des gebogenen distalen Endes (19) ist.

9. Der Katheter nach Anspruch 8, worin die zweiten und dritten Lichtungen (15, 16) mit Abstand angeordnet sind.

10. Der Katheter nach Anspruch 1, worin der Durchmesser des Rohrs (11) an dem distalen Ende reduziert ist.

11. Der Katheter nach Anspruch 1, worin das gebogene distale Ende (19) des Rohrs (11) einen geringeren Durchmesser als der Bereich des Rohrs (11) aufweist, der sich zwischen dem gebogenen distalen Ende und dem proximalen Ende erstreckt.

## Revendications

1. Cathéter de veine centrale (10) destiné à prélever, injecter ou contrôler les fluides à l'intérieur du système vasculaire central, comprenant :
un tube plastique souple d'une seule pièce (11) d'un diamètre approprié pour l'introduction percutanée dans le système vasculaire, ce tube ayant au moins une lumière (14), l'extrémité distale (19) du tube (11) étant recourbée d'au moins 220° de façon à présenter un bord d'attaque émoussé dans l'utilisation et en l'absence d'obstruction par un guide-fil.

2. Cathéter destiné à prélever, injecter ou contrôler les fluides selon la revendication 1, dans lequel l'extrémité distale (19) du tube (11) est conique et au moins une lumière (14) s'étend complètement à travers le tube, dans lequel ce tube a une pointe distale (17) et dans lequel une portion (21) de l'extrémité distale (19) contiguë à cette pointe distale (27) n'est pas recourbée.

3. Cathéter destiné à prélever, injecter ou contrôler les fluides selon la revendication 1 ou 2, dans lequel le tube (11) définit une seconde lumière (15) séparée d'au moins une lumière (14), ce tube (11) définissant également un premier orifice latéral (23) espacé de l'extrémité distale et la seconde lumière (15) aboutissent dans et étant en communication avec le premier orifice latéral (23).

4. Cathéter destiné à prélever, injecter ou contrôler les fluides selon la revendication 3, dans lequel le tube définit une troisième lumière (16) séparée d'au moins une lumière (14) et à partir de la seconde lumière (15), le tube (11) définissant également un second orifice latéral (24) espacé de l'extrémité distale (19) et à partir du premier orifice latéral (23) et la troisième lumière (16) aboutissent dans et étant en communication avec le second orifice latéral (24).

5. Cathéter destiné à prélever, injecter ou contrôler les fluides selon les revendications 1, 2, 3 ou 4, dans lequel l'extrémité distale (19) du tube (11) s'enroule d'au moins 235° en l'absence d'obstruction par le guide-fil.

6. Cathéter selon la revendication 1, dans lequel la première lumière est disposée à l'extrémité de la pointe distale (17).

7. Cathéter selon la revendication 6, dans lequel l'orifice latéral (23) de la seconde lumière (15) est proximal de l'extrémité, distale recourbée (19).

8. Cathéter selon la revendication 7, dans lequel l'orifice latéral (24) de le troisième lumière (16) est proximal de l'extrémité distale recourbée (19).

9. Cathéter selon la revendication 8, dons lequel les seconde et troisième lumières (15, 16) sont espacées entre elles.

10. Cathéter selon la revendication 1, dans lequel le diamètre du tube (11) est réduit au niveau de l'extrémité distale.

11. Cathéter selon la revendication 1, dans lequel l'extrémité distale recourbée (19) du tube (11) possède un diamètre plus petit que la portion du tube (11) qui s'étend entre l'extrémité distale recourbée et l'extrémité proximale.
